(19) Europäisches Patentamt
European Patent Office
Office européen des brevets

(11) **EP 3 442 564 B1**

(12) **EUROPEAN PATENT SPECIFICATION**

(45) Date of publication and mention
of the grant of the patent:
**06.12.2023 Bulletin 2023/49**

(21) Application number: **17781637.8**

(22) Date of filing: **12.04.2017**

(51) International Patent Classification (IPC):
***A61K 38/54*** *(2006.01)*    ***A61K 38/48*** *(2006.01)*
***A61P 35/00*** *(2006.01)*

(52) Cooperative Patent Classification (CPC):
**A61K 38/4826; A61K 38/54; A61P 35/00;**
**C12Y 304/21001; C12Y 304/21004**

(86) International application number:
**PCT/AU2017/050323**

(87) International publication number:
**WO 2017/177270 (19.10.2017 Gazette 2017/42)**

(54) **COMPOSITION OF PROENZYMES FOR CANCER TREATMENT**

ZUSAMMENSETZUNG AUS PROENZYMEN ZUR KREBSBEHANDLUNG

COMPOSITION DE PROENZYMES POUR LE TRAITEMENT DU CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.04.2016 US 201662321370 P**

(43) Date of publication of application:
**20.02.2019 Bulletin 2019/08**

(73) Proprietor: **Propanc Pty Ltd**
**Camberwell, Victoria 3124 (AU)**

(72) Inventors:
• **KENYON, Julian**
**Camberwell, Victoria 3124 (AU)**
• **BRANDT, Ralf**
**Camberwell, Victoria 3124 (AU)**

(74) Representative: **Weickmann & Weickmann
PartmbB
Postfach 860 820
81635 München (DE)**

(56) References cited:
WO-A1-2011/047434    WO-A1-2015/070828
WO-A1-2017/127892    US-A- 4 514 388
US-A- 5 858 357

• **PERAN MACARENA ET AL: "A formulation of
pancreatic proenzymes provides potent
antitumour efficacy: a pilot study focused on
pancreatic and ovarian cancer", SCIENTIFIC
REPORTS,, vol. 7, 25 October 2017 (2017-10-25),
XP002790107, ISSN: 2045-2322**
• **PERAN ET AL.: 'In vitro Treatment of Carcinoma
Cell Lines With Pancreatic (Pro)enzymes
Suppresses the EMT Programme and Promotes
Cell Differentiation' CELLULAR ONCOLOGY vol.
36, 2013, pages 289 - 301, XP055380387**
• **NOVAK J.F. ET AL.: 'Proenzyme Therapy of
Cancer' ANTICANCER RESEARCH vol. 25, 2005,
pages 1157 - 1178, XP008156400**
• **KAISEROVA P. ET AL.: 'Proenzyme Therapy of
Sarcoma S-180 and Melanoma B16-F10'
JOURNAL OF APPLIED BIOMEDICINE vol. 12,
2014, pages 39 - 47, XP055380384**

**Description**

**Field of the invention**

The present invention relates to compositions, methods, uses and kits for treating cancer.

**Background of the invention**

The use of proteases in treating cancer has been suggested for some time. Initially fresh pancreatic enzyme extracts were contemplated as a possible cancer therapy and some successful experiments were conducted with Jersen's mouse sarcoma model. After injecting the mouse with the protease enzyme trypsin, a regression of tumours was observed. The results obtained produced great interest, and crude enzyme extracts prepared from sheep pancreas were used to treat human cancer patients to reduce tumour progression and prolong survival time.

The use of proenzymes (inactive precursor form of enzymes) has been used to try to overcome problems encountered with the oral administration of enzymes with mixed results. A proenzyme mixture including trypsinogen, which is the proenzyme form of the serine protease inhibitor trypsin, has been shown to be useful in treating carcinomas and believed to be selectively activated at the surface of tumour cells. The mechanism of action of trypsin is believed to occur by way of proteolysis of the tumour cells. A composition including chymotrypsinogen and trypsinogen has been shown to be effective in assays for cancer, including pancreatic cancer and colon cancer (WO 2011/047434). International application WO2017/127892, which was published after the priority date of the present application, discloses a composition comprising chymotrypsinogen and trypsinogen in a weight ratio in the range of 1:1 to 10:1.

However, there exists a need to provide new or improved cancer treatments.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

**Summary of the invention**

The invention provides a composition for treating cancer in a subject comprising chymotrypsinogen and trypsinogen, wherein the weight ratio of chymotrypsinogen : trypsinogen is equal to or greater than 2:1 but less than 4:1 (i.e. there is at least two times but less than 4 times the amount of chymotrypsinogen compared with trypsinogen), and wherein the composition does not comprise amylase. Preferably, the weight ratio of chymotrypsinogen : trypsinogen is about 2:1.

The present document also describes a unit dose composition comprising chymotrypsinogen and trypsinogen, wherein the unit dose is adapted to administer chymotrypsinogen in an amount of greater than, or equal to, 6mg or any other amount as described herein, wherein the weight ratio of chymotrypsinogen : trypsinogen is equal to or greater than 2:1 but less than 4:1. Preferably, the weight ratio of chymotrypsinogen : trypsinogen is about 2:1. Preferably, chymotrypsinogen is in an amount of greater than, or equal to, 9mg, 15mg, 24mg, 72mg, 210mg, 600mg, 1200mg, or 2400mg.

The present document also describes a unit dose composition comprising chymotrypsinogen and trypsinogen, wherein the unit dose is adapted to administer trypsinogen in an amount of greater than, or equal to, 1 mg, or any other amount as described herein, wherein the weight ratio of chymotrypsinogen : trypsinogen is equal to or greater than 2:1 but less than 4:1. Preferably, the weight ratio of chymotrypsinogen : trypsinogen is about 2:1. Preferably, trypsinogen is in an amount of greater than or equal to 1.5mg, 3mg, 3.5mg, 12 mg, 36mg, 90mg, 180mg or 360mg.

In any aspect, the composition comprises or consists of chymotrypsinogen and trypsinogen, wherein the amount of chymotrypsinogen is equal to, greater than, 1.5mg/kg, 2mg/kg, 3.5mg/kg, 5mg/kg, 15mg/kg, 20mg/kg, 40mg/kg, 45mg/kg, 135mg/kg, 250mg/kg or 500mg/kg. The amount of chymotrypsinogen and trypsinogen may be equal to, or greater than, any mg/kg value described herein.

In any aspect, the composition for treating cancer in a human comprises or consists of chymotrypsinogen and trypsinogen, wherein the amount of chymotrypsinogen is equal to, or greater than, 0.1 mg/kg, 0.15mg/kg, 0.25mg/kg, 0.4mg/kg, 1.2mg/kg, 3.5mg/kg, 10mg/kg, 20mg/kg or 40 mg/kg. The amount of chymotrypsinogen and trypsinogen may be equal to, or greater than, any mg/kg value described herein.

In any aspect, the composition comprises or consists of chymotrypsinogen and trypsinogen, wherein the amount of trypsinogen is equal to, or greater than, 0.25 mg/kg, 0.4mg/kg, 0.6 mg/kg, 0.8mg/kg, 2mg/kg, 2.5mg/kg, 3mg/kg, 5mg/kg, 7mg/kg, 8mg/kg, 20 mg/kg, 40mg/kg or 80mg/kg. The amount of chymotrypsinogen and trypsinogen may be equal to, or greater than, any mg/kg value described herein.

In any aspect, the composition for treating cancer in a human comprises or consists of chymotrypsinogen and trypsinogen, wherein the amount of trypsinogen is equal to, or greater than, 0.02mg/kg, 0.03mg/kg, 0.05mg/kg, 0.06mg/kg, 0.2mg/kg, 0.6mg/kg, 1.5mg/kg, 3mg/kg or 6mg/kg. The amount of chymotrypsinogen and trypsinogen may be equal to, or greater than, any mg/kg value described herein.

In any aspect, the pharmaceutical composition comprises, consists of, or is adapted to administer, chymotrypsinogen and trypsinogen, wherein the amount of chymotrypsinogen is equal to, or greater than, 1.5mg/kg, 2mg/kg, 3.5mg/kg, 5mg/kg, 15mg/kg, 20mg/kg, 40mg/kg, 45mg/kg, 135mg/kg, 250mg/kg or 500mg/kg. The amount of chymotrypsinogen and trypsinogen may be equal to, or greater than, any mg/kg value described herein.

In any aspect, the pharmaceutical composition for human use comprises, consists of, or is adapted to administer chymotrypsinogen and trypsinogen, wherein the amount of chymotrypsinogen is equal to, or greater than, 0.1mg/kg, 0.15mg/kg, 0.25mg/kg, 0.4mg/kg, 1.2mg/kg, 3.5mg/kg, 10mg/kg, 20mg/kg or 40 mg/kg. The amount of chymotrypsinogen may be equal to, or greater than, any mg/kg value described herein.

In any aspect, the pharmaceutical composition comprises, consists of, or is adapted to administer, chymotrypsinogen and trypsinogen, wherein the amount of trypsinogen administered is equal to, or greater than, 0.25 mg/kg, 0.4mg/kg, 0.6 mg/kg, 0.8mg/kg, 2mg/kg, 2.5mg/kg, 3mg/kg, 5mg/kg, 7mg/kg, 8mg/kg, 20 mg/kg, 40mg/kg or 80mg/kg. The amount of trypsinogen may be equal to, or greater than, any mg/kg value described herein.

In any aspect, the pharmaceutical composition for human use comprises, consists of, or is adapted to administer chymotrypsinogen and trypsinogen, wherein the amount of trypsinogen administered is equal to, or greater than, 0.02mg/kg, 0.03mg/kg, 0.05mg/kg, 0.06mg/kg, 0.2mg/kg, 0.6mg/kg, 1.5mg/kg, 3mg/kg or 6mg/kg. The amount of trypsinogen may be equal to, or greater than, any mg/kg value described herein.

While the present invention relates to a composition for use in treating cancer, the respective method of treatment is not included in the scope of the invention.

In any method of treatment, the amount of chymotrypsinogen administered in a single dose or in multiple doses over the period of a 24 hour period is greater than 0.1mg/kg but less than 500 mg/kg. Preferably, the chymotrypsinogen is administered in an amount of greater than 1mg/kg.

In any method of treatment, the amount of trypsinogen administered in a single dose or in multiple doses over the period of a 24 hour period is at least greater than 0.02 mg/kg but less than 90 mg/kg. Preferably, the trypsinogen is administered in an amount of greater than 0.2 mg/kg.

In any method of treatment, the administration of the amount of trypsinogen and chymotrypsinogen does not result in any clinically observable adverse event in the subject 1 week after administration, 1 day after administration, or preferably 1 hour after administration. The clinically observable adverse event may be any one or more of weight loss, reddening at site of injection and behavioural changes, or any other event described herein.

The present document also describes a kit for treating cancer comprising at least one dosage unit, wherein the dosage unit comprises chymotrypsinogen, trypsinogen and a pharmaceutically acceptable diluent, excipient or carrier, wherein the dosage unit is adapted to administer chymotrypsinogen and trypsinogen equal to, or greater than, any amount or mg/kg value described herein.

Optionally the kit also includes written instructions directing the user to administer a dosage unit of chymotrypsinogen in an amount of greater than 1mg/kg or any other amount as described herein.

Optionally the kit also includes written instructions directing the user to administer a dosage unit of trypsinogen in an amount of greater than 0.2 mg/kg, or any other amount as described herein.

The compositions for use of the invention are useful for treating cancers and metastatic carcinomas including pancreatic cancer, oesophageal cancer, colon cancer, bowel cancer, uterine cancer, prostate cancer, ovarian cancer, brain cancer, stomach cancer, breast cancer, liver cancer, kidney cancer, malignant melanoma, fibrosarcoma or lung cancer. Preferably, the cancer is pancreatic cancer, colon cancer or ovarian cancer. More preferably, the cancer is pancreatic cancer. Preferably, the brain cancer is glioblastoma.

Medical treatment may further comprise the step of identifying a subject having, or at risk of developing, cancer. Preferably, the cancer is any one described herein.

In any aspect of the invention, the composition does not contain amylase, i.e. the composition is amylase-free.

In any aspect described herein the amount of chymotrypsinogen administered may be greater than, or equal to, 1mg/kg, 1.5mg/kg, 2mg/kg, 3.5mg/kg, 5mg/kg, 15mg/kg, 20mg/kg, 40mg/kg, 45mg/kg, 135mg/kg, 250mg/kg or 500mg/kg. The chymotrypsinogen administered may be equal to, or greater than, any mg/kg value described herein.

In any aspect described herein the amount of trypsinogen administered may be greater than, or equal to, 0.2mg/kg, 0.25 mg/kg, 0.4mg/kg, 0.6 mg/kg, 0.8mg/kg, 2mg/kg, 2.5mg/kg, 3mg/kg, 5mg/kg, 7mg/kg, 8mg/kg, 20 mg/kg, 40mg/kg or 80mg/kg. The trypsinogen administered may be equal to, or greater than, any mg/kg value described herein.

In any aspect described herein the amount of chymotrypsinogen administered to a human may be greater than, or equal to, 0.1mg/kg, 0.15mg/kg, 0.25mg/kg, 0.4mg/kg, 1.2mg/kg, 3.5mg/kg, 10mg/kg, 20mg/kg or 40 mg/kg. The amount of chymotrypsinogen may be equal to, or greater than, any mg/kg value described herein.

In any aspect described herein the amount of trypsinogen administered to a human may be greater than, or equal to, 0.02mg/kg, 0.03mg/kg, 0.05mg/kg, 0.06mg/kg, 0.2mg/kg, 0.6mg/kg, 1.5mg/kg, 3mg/kg or 6mg/kg. The amount of trypsinogen may be equal to, or greater than, any mg/kg value described herein.

Preferably, in any aspect described herein the amount of chymotrypsinogen administered may be in the range of 1mg/kg to 41mg/kg, 1.5mg/kg to 500mg/kg, 2mg/kg to 250mg/kg, 3.5mg/kg to 135mg/kg, 5mg/kg or 15mg/kg to 45mg/kg. The chymotrypsinogen administered may be in a range of between any two mg/kg values described herein.

Preferably, in any aspect described herein the amount of trypsinogen administered may be greater than 0.2mg/kg to 7mg/kg, 0.25 mg/kg to 80mg/kg, 0.4mg/kg to 40mg/kg, 0.6 mg/kg to 20 mg/kg, 0.8mg/kg to 8mg/kg, or 2.5mg/kg to 8mg/kg. The trypsinogen administered may be in a range of between any two mg/kg values described herein.

In any composition for use of the invention above, the composition may be adapted to administer the relevant mg, or mg/kg, of chymotrypsinogen and trypsinogen to the subject.

Chymotrypsinogen and trypsinogen can be administered intravenously, intraperitoneally, subcutaneously or intramuscularly.

Chymotrypsinogen and trypsinogen may be administered simultaneously or sequentially. In relation to sequential administration, chymotrypsinogen may be administered first then trypsinogen, or trypsinogen first and then chymotrypsinogen.

As used herein, except where the context requires otherwise, the term "comprise" and variations of the term, such as "comprising", "comprises" and "comprised", are not intended to exclude further additives, components, integers or steps.

Further aspects of the present invention and further embodiments of the aspects described in the preceding paragraphs will become apparent from the following description, given by way of example and with reference to the accompanying drawings.

**Brief description of the drawings**

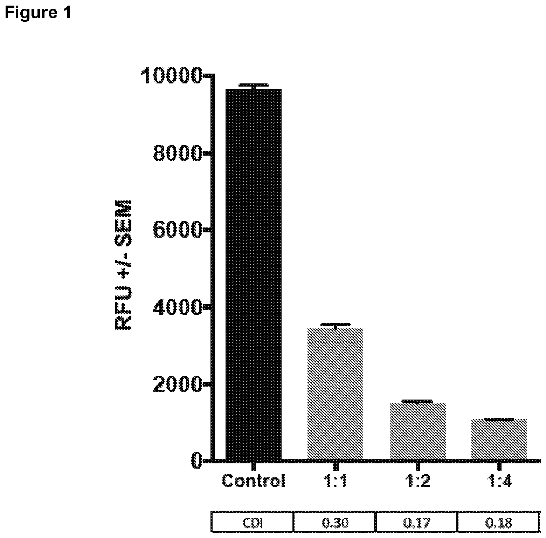

Figure 1: The Effect of Trypsinogen and Chymotrypsinogen in Combination on the Growth of A2780 human ovary tumour cells. Combination assays of Trypsinogen and Chymotrypsinogen were performed at ratios of 1:1, 1:2 and 1:4 based on the previously determined $IC_{50}$ of Trypsinogen (3.174mg/ml). Coefficient of daily interaction (CDI) values were calculated as described.

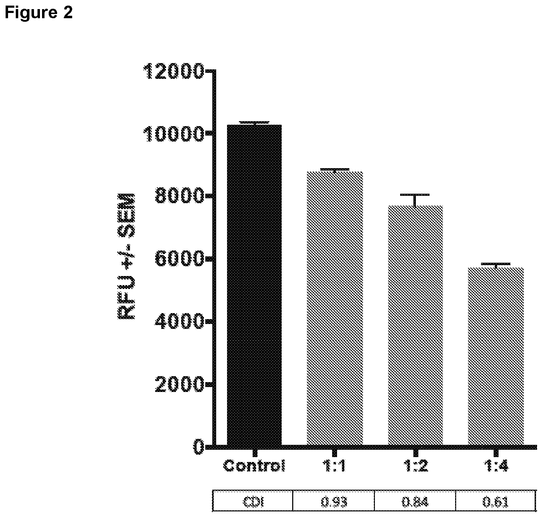

Figure 2: The Effect of Trypsinogen and Chymotrypsinogen in Combination on the Growth of ACHN human kidney tumour cells. Combination assays of Trypsinogen and Chymotrypsinogen were performed at ratios of 1:1, 1:2 and 1:4 based on the previously determined IC50 of Trypsinogen (2.984mg/ml). CDI values were calculated as described.

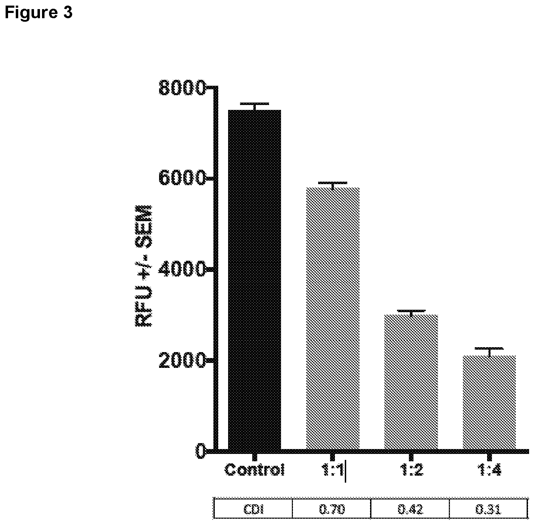

Figure 3: The Effect of Trypsinogen and Chymotrypsinogen in Combination on the Growth of C8161.9 human melanoma cells. Combination assays of Trypsinogen and Chymotrypsinogen were performed at ratios of 1:1, 1:2 and 1:4 based on the previously determined IC50 of Trypsinogen (3.917mg/ml). CDI values were calculated as described.

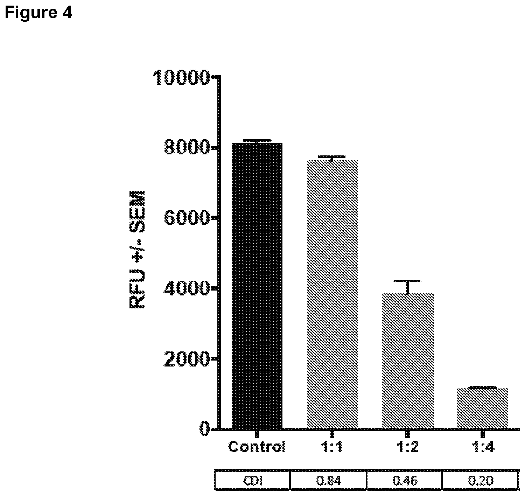

Figure 4: The Effect of Trypsinogen and Chymotrypsinogen in Combination on the Growth of DAOY human brain tumour cells. Combination assays of Trypsinogen and Chymotrypsinogen were performed at ratios of 1:1, 1:2 and 1:4 based on the previously determined IC50 of Trypsinogen (2.654mg/ml). CDI values were calculated as described.

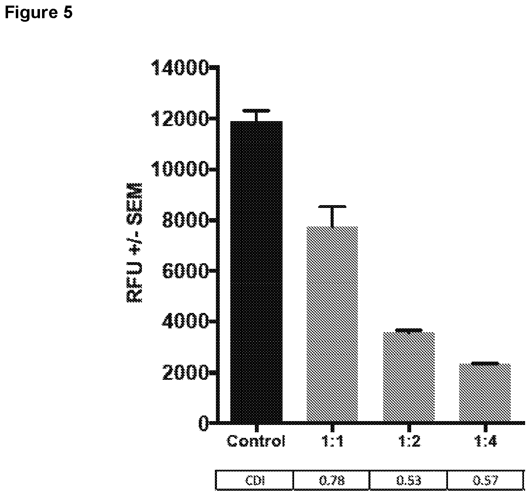

Figure 5: The Effect of Trypsinogen and Chymotrypsinogen in Combination on the Growth of DU 145 human prostate tumour cells. Combination assays of Trypsinogen and Chymotrypsinogen were performed at ratios of 1:1, 1:2 and 1:4 based on the previously determined IC50 of Trypsinogen (3.843mg/ml). CDI values were calculated as described.

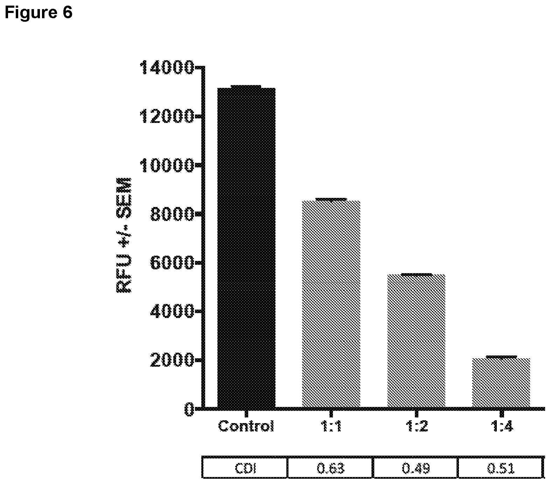

Figure 6: The Effect of Trypsinogen and Chymotrypsinogen in Combination on the Growth of G-361 human melanoma cells. Combination assays of Trypsinogen and Chymotrypsinogen were performed at ratios of 1:1, 1:2 and 1:4 based on the previously determined IC50 of Trypsinogen (16.05mg/ml). CDI values were calculated as described.

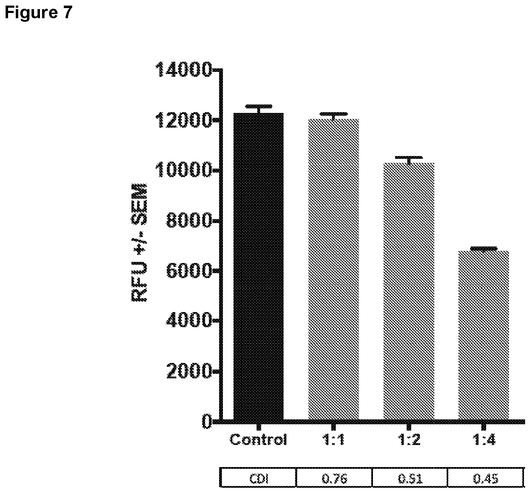

Figure 7: The Effect of Trypsinogen and Chymotrypsinogen in Combination on the Growth of HCT 116 human colorectal tumour cells. Combination assays of Trypsinogen and Chymotrypsinogen were performed at ratios of 1:1, 1:2 and 1:4 based on the previously determined IC50 of Trypsinogen (16.43mg/ml). CDI values were calculated as described.

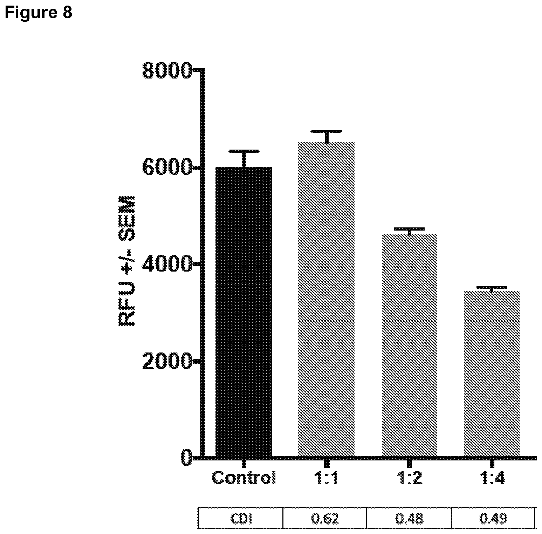

Figure 8: The Effect of Trypsinogen and Chymotrypsinogen in Combination on the Growth of HCT-15 human colorectal tumour cells. Combination assays of Trypsinogen and Chymotrypsinogen were performed at ratios of 1:1, 1:2 and 1:4 based on the previously determined IC50 of Trypsinogen (2.977mg/ml). CDI values were calculated as described.

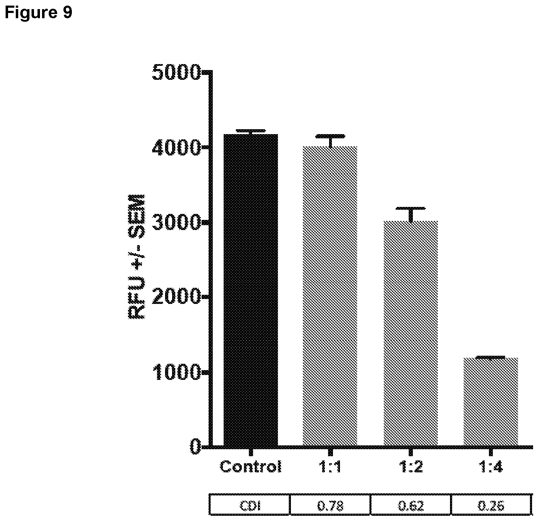

**Figure 9:** The Effect of Trypsinogen and Chymotrypsinogen in Combination on the Growth of Hep3B2.1-7 human liver tumour cells. Combination assays of Trypsinogen and Chymotrypsinogen were performed at ratios of 1:1, 1:2 and 1:4 based on the previously determined IC50 of Trypsinogen (2.483mg/ml). CDI values were calculated as described.

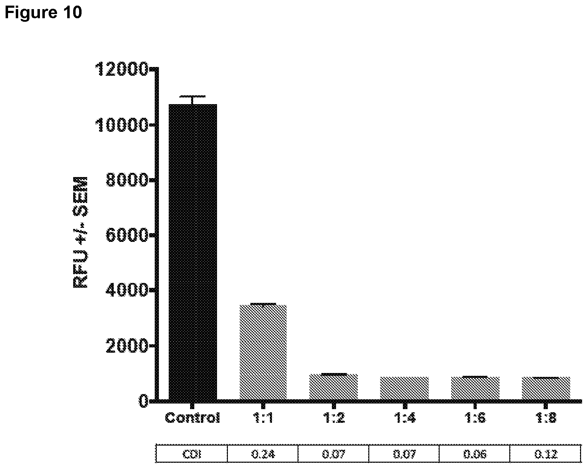

**Figure 10:** The Effect of Trypsinogen and Chymotrypsinogen in Combination on the Growth of HT-1080 human fibrosarcoma cells. Combination assays of Trypsinogen and Chymotrypsinogen were performed at ratios of 1:1, 1:2, 1:4, 1:6 and 1:8 based on the previously determined IC50 of Trypsinogen (4.224mg/ml). CDI values were calculated as described.

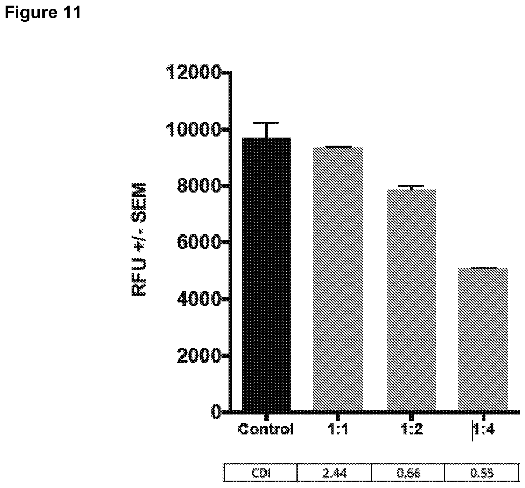

**Figure 11:** The Effect of Trypsinogen and Chymotrypsinogen in Combination on the Growth of HT-29 human colorectal tumour cells. Combination assays of Trypsinogen and Chymotrypsinogen were performed at ratios of 1:1, 1:2 and 1:4 based on the previously determined IC50 of Trypsinogen (15.12mg/ml). CDI values were calculated as described.

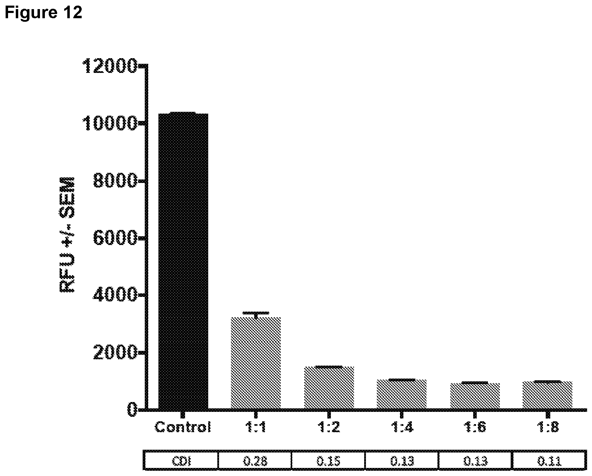

**Figure 12:** The Effect of Trypsinogen and Chymotrypsinogen in Combination on the Growth of HuH-7 human liver tumour cells. Combination assays of Trypsinogen and Chymotrypsinogen were performed at ratios of 1:1, 1:2, 1:4, 1:6 and 1:8 based on the previously determined IC50 of Trypsinogen (3.934mg/ml). CDI values were calculated as described.

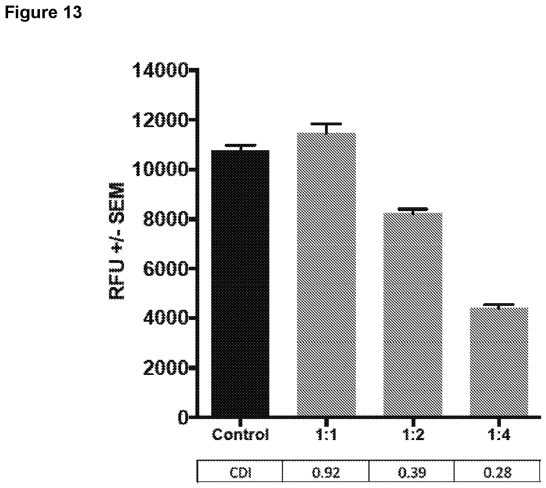

**Figure 13:** The Effect of Trypsinogen and Chymotrypsinogen in Combination on the Growth of MCF-7 human breast tumour cells. Combination assays of Trypsinogen and Chymotrypsinogen were performed at ratios of 1:1, 1:2 and 1:4 based on the previously determined IC50 of Trypsinogen (16.57mg/ml). CDI values were calculated as described.

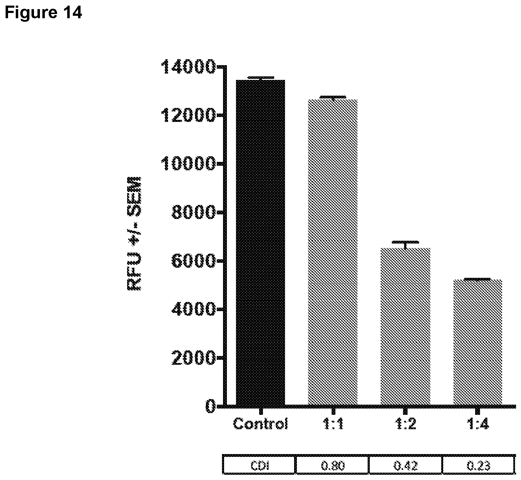

**Figure 14:** The Effect of Trypsinogen and Chymotrypsinogen in Combination on the Growth of MES-SA human uterine tumour cells. Combination assays of Trypsinogen and Chymotrypsinogen were performed at ratios of 1:1, 1:2 and 1:4 based on the previously determined IC50 of Trypsinogen (7.806mg/ml). CDI values were calculated as described.

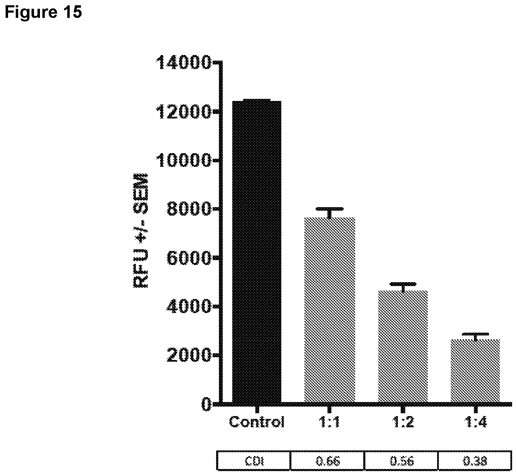

**Figure 15:** The Effect of Trypsinogen and Chymotrypsinogen in Combination on the Growth of NCI-H460 human lung tumour cells. Combination assays of Trypsinogen and Chymotrypsinogen were performed at ratios of 1:1, 1:2 and 1:4 based on the previously determined IC50 of Trypsinogen (4.028mg/ml). CDI values were calculated as described.

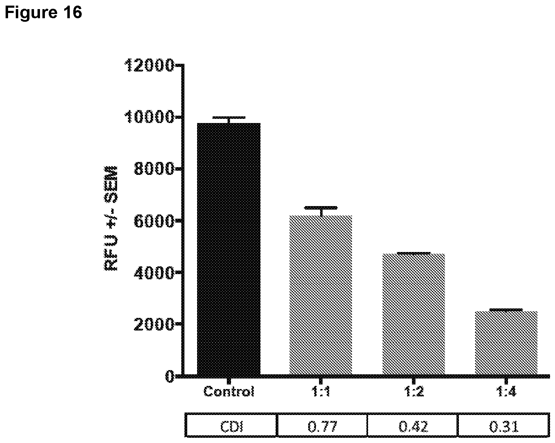

**Figure 16:** The Effect of Trypsinogen and Chymotrypsinogen in Combination on the Growth of PC-3 human prostate tumour cells. Combination assays of Trypsinogen and Chymotrypsinogen were performed at ratios of 1:1, 1:2 and 1:4 based on the previously determined IC50 of Trypsinogen (17.46mg/ml). CDI values were calculated as described.

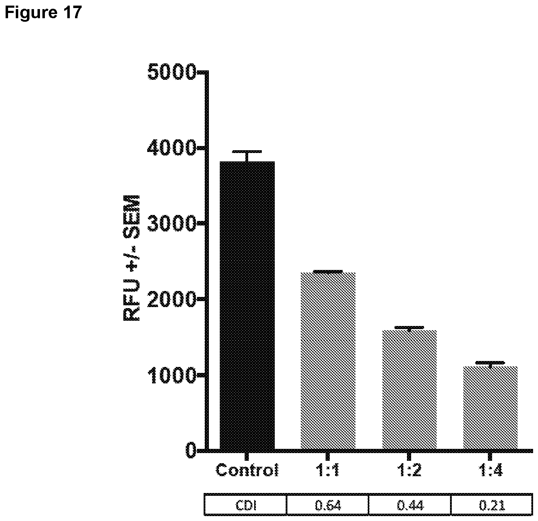

**Figure 17:** The Effect of Trypsinogen and Chymotrypsinogen in Combination on the Growth of SK-OV-3 human ovary tumour cells. Combination assays of Trypsinogen and Chymotrypsinogen were performed at ratios of 1:1, 1:2 and 1:4 based on the previously determined IC50 of Trypsinogen (8.662mg/ml). CDI values were calculated as described.

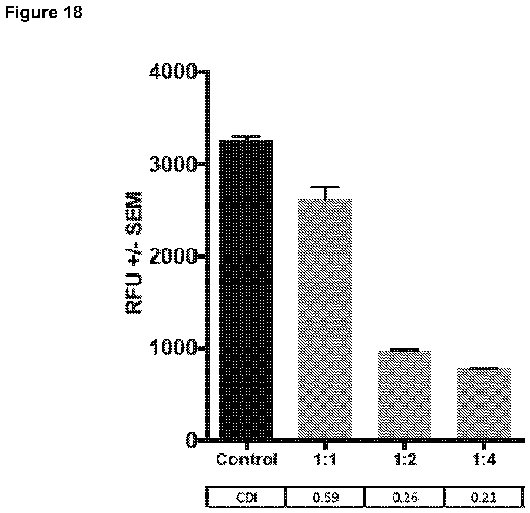

**Figure 18:** The Effect of Trypsinogen and Chymotrypsinogen in Combination on the Growth of SNB-19 human brain tumour cells. Combination assays of Trypsinogen and Chymotrypsinogen were performed at ratios of 1:1, 1:2 and 1:4 based on the previously determined IC50 of Trypsinogen (3.945mg/ml). CDI values were calculated as described.

## Detailed description of the embodiments

Reference will now be made in detail to certain embodiments of the invention. While the invention will be described in conjunction with the embodiments, it will be understood that the intention is not to limit the invention which is defined by the claims.

For purposes of interpreting this specification, terms used in the singular will also include the plural and vice versa.

In one aspect, the present invention is based on the surprising finding that chymotrypsinogen and trypsinogen can be administered at a weight ratio of equalt to or greater than 2:1 but less than 4:1. Surprisingly, significant growth inhibition of a wide range of tumour cells is observed without the need for 4 times, or more, the weight amount of chymotrypsinogen relative to trypsinogen. Prior publications have suggested a weight ratio of chymotrypsinogen and trypsinogen of 6:1 is optimal but the inventors now surprisingly identified that a significant inhibitory effect on cancer cell growth is achieved with much less chymotrypsinogen present relative to trypsinogen.

In another aspect, the present invention is based on the finding that chymotrypsinogen and trypsinogen is effective against fibrosarcoma.

Chymotrypsinogen (which may be abbreviated to 'C' herein) is a proenzyme form of the enzyme chymotrypsin, which preferentially cleaves proteins at the following amino acids: tyrosine, tryptophan, phenylalanine and leucine. Chymotrypsin may be referred to or includes chymotrypsin A, chymotrypsin B (including B1 and B2 forms), chymotrypsin C, α-chymar ophth, avazyme, chymar, chymotest, enzeon, quimar, quimotrase, α-chymar, α-chymotrypsin A, α-chymotrypsin. Chymotrypsin C can be formed from pig chymotrypsinogen C or from cattle subunit II of procarboxypeptidase A, and preferentially cleaves proteins at the following amino acids: tyrosine, tryptophan, phenylalanine, leucine, methionine, glutamine, and asparagine. Chymotrypsinogen includes chymotrypsinogen B1 and chymotrypsinogen B2.

Trypsinogen (which may be abbreviated to 'T' herein) is a proenzyme form of trypsin, which preferentially cleaves proteins at arginine and lysine. Trypsin may be referred to or include α-trypsin, β-trypsin, cocoonase, parenzyme, parenzymol, tryptar, trypure, pseudotrypsin, tryptase, tripcellim, sperm receptor hydrolase β-trypsin can be formed from trypsinogen by cleavage of one peptide bond. Further peptide bond cleavages produce α and other iso-forms. Multiple cationic and anionic trypsins can be isolated from the pancreas of many vertebrates and from lower species including crayfish, insects (cocoonase) and microorganisms (Streptomyces griseus). In normal processes during digestion, inactive trypsinogen is activated by enteropeptidase present in intestinal mucosa to form the enzyme trypsin, which being a serine protease then acts to cleave the peptide bonds on the carboxyl side of basic amino acids/proteins.

The trypsinogen and chymotrypsinogen used in any aspect of the invention may be isolated, purified, substantially purified, recombinant or synthetic.

The proenzymes trypsinogen and chymotrypsinogen may be precursors of the enzymes selected from chymotrypsin classes 3.4.21.1 or 3.4.21.2 or trypsin from class 3.4.21.4, or selected from any other suitable source (classes grouped according to the classification of the Nomenclature Committee of the International Union of Biochemistry and Molecular Biology). These enzymes are commercially available and may be of human, bovine or porcine origin.

The present invention includes human conversions for all amounts in mg/kg referred to herein based on human body weights of 50, 60, 70, 80, 90, 100 or more kg and body surface area of 1.1, 1.2, 1.3, 1.4, 1.5, 1.6, 1.7, 1.8 or more m2. Specifically, amounts per kg for human administration, and methods for calculation, are referred to in Example 5.

As mentioned, the proenzyme form essentially provides an inactivated form of the enzyme that becomes activated in situ (e.g in vivo or in vitro activation). For example, activation of the proenzyme (conversion of proenzyme to active enzyme) may occur on contact with the surface of the tumour cell. It is believed that the proenzymes trypsinogen and chymotrypsinogen are selectively activated into the enzymes trypsin and chymotrypsin on contact with tumour cells and not on contact with healthy cells. The use of proenzymes reduces problems associated with providing, in situ, an active enzyme, such as undesirable reactions or inactivation of the enzyme before reaching an intended target of a tumour cell.

In relation to tumour cells, protease enzymes can act to break down the cell wall of malignant cells by cleaving the amide bonds present in peptide chains of the cell walls (proteolysis). It is also understood that protease inhibitors, which are present in non-malignant cells and inhibit or reduce the effect of enzymes in breaking down cell walls, are absent in malignant tumour cells. In addition to providing proteolytic activity, the protease proenzymes can upregulate the expression of β-catenin and E-cadherin in tumour cells. Cell-to-cell adhesion is facilitated by complex formation or bonding between β-catenin and E-cadherin at the cell surface, and therefore increased expression of β-catenin and E-cadherin leads to enhanced cell-to-cell adhesion and thereby reducing metastasis of tumour cells. The protease proenzymes may also provide other cellular activity such as increased immunorecognition or differentiation.

A significant adverse event, experience or reaction is any untoward medical occurrence that at any dose: results in death, is life-threatening (places the subject at immediate risk of death), requires in subject/subject hospitalization or prolongation of existing hospitalization, results in persistent or significant disability, incapacity, or is a congenital anomaly/birth defect.

A clinically observable adverse event is any untoward medical occurrence in a subject or clinical investigation subject administered a pharmaceutical product. A clinically observable adverse event may include any one of the events or observations described herein, particularly in the Examples. Typically, the period of observation is about 1 week after administration, about 1 day after administration, or preferably about 1 hour after administration. The period of observation may be the time between administration of doses.

"Treating" or "treatment" refers to both therapeutic treatment and prophylactic or preventative measures, wherein the aim is to prevent, ameliorate, reduce or slow down (lessen) cancer or the spread (metastasis) thereof.

With the composition for use according to the invention, one or more of the following effects may be observed: reduction in the reoccurrence of malignant tumours, reduction in metastasis of malignant tumours, reduction in number or size of tumours, differentiation of tumour cells, expression of β-catenin and E-cadherin in malignant tumours to facilitate cell-to-cell adhesion and reduction in metastasis, reduction in tumour cells ability to prevent immunorecognition.

"Preventing", "prevention", "preventative" or "prophylactic" refers to keeping from occurring, or to hinder, defend from, or protect from the occurrence of a condition, disease, disorder, or phenotype, including an abnormality or symptom. A subject in need of prevention may be prone to develop the condition.

The term "ameliorate" or "amelioration" refers to a decrease, reduction or elimination of a condition, disease, disorder, or phenotype, including an abnormality or symptom. A subject in need of treatment may already have the condition, or may be prone to have the condition or may be one in whom the condition is to be prevented.

The "subject" includes a mammal. The mammal may be a human, or may be a domestic, zoo, or companion animal. While it is particularly contemplated that the compositions for use of the invention are suitable for medical treatment of humans, they are also applicable to veterinary treatment, including treatment of companion animals such as dogs and cats, and domestic animals such as horses, cattle and sheep, or zoo animals such as felids, canids, bovids, and ungulates. A subject may be afflicted with cancer or other disorder, or may not be afflicted with cancer or other disorder (i.e., free of detectable disease).

The typical body weight of a human subject may be greater than, or equal to, 60, 65, 70, 75, 80, 85, 90, 95, 100, 105 or 110kg.

The term "therapeutically effective amount" refers to an amount of composition, or agent or compound in the composition, capable of treating, preventing or ameliorating cancer or the spread (metastasis) thereof. A therapeutically effective amount may be determined empirically and in a routine manner in relation to treating cancer, and will result in increased life expectancy.

As used herein "adapted to administer" refers to a composition that has the capacity to administer trypsinogen and chymotrypsinogen at the specified amount or concentration in a single dose or multiple doses.

As described herein, treating cancer may include treatment of neoplasms and related conditions, cancers, tumours, malignant and metastatic conditions. Tissues and organs associated with solid tumours and metastases which can be treated with a composition for use of the invention include, but are not limited to, biliary tract, bladder, blood, brain, breast, cervix, colon, endometrium, oesophagus, head, neck, kidney, larynx, liver, lung, medulla, melanin, ovarian, pancreas, prostate, rectum, renal, retina, skin, stomach, testes, thyroid, urinary tract, and uterus.

The compositions for use of the invention are useful for treating cancers and metastatic carcinomas of the following types: pancreatic cancer, oesophageal cancer, colon cancer, bowel cancer, prostate cancer, ovarian cancer, stomach cancer, breast cancer, malignant melanoma or lung cancer. Preferably, the cancer is pancreatic cancer, colon cancer or ovarian cancer. More preferably, the cancer is pancreatic cancer. The metastatic potential of the carcinoma may be low, moderate or high.

The compositions for use of the invention may provide a multiple effect approach to treating cancer, for example by increasing in tumour cells apoptosis, cell-to-cell adhesion, differentiation and immunogenicity (targeting and removal by immune system). It is therefore beneficial to conduct treatment in the absence of any other treatments that may suppress or harm the immune system.

Compositions for use of the invention can be supplemented by other conventional anti-cancer therapeutic approaches directed to treatment or prevention of proliferative disorders (e.g., tumour). For example, compositions for use of the invention can be used in prophylactic cancer prevention, prevention of cancer recurrence and metastases after surgery, and as an adjuvant of other conventional cancer therapy.

The compositions for use of the invention may be formulated, for example, by employing conventional solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation.

The compositions for use of the invention may be administered by any suitable means, for example, orally, such as in the form of tablets, capsules, granules or powders; sublingually; buccally; parenterally, such as by subcutaneous, intravenous, intramuscular, or intracisternal injection or infusion techniques (e.g., as sterile injectable aqueous or non-aqueous solutions or suspensions); nasally such as by inhalation spray; topically, such as in the form of a cream or ointment; or rectally such as in the form of suppositories; in dosage unit formulations containing non-toxic, pharmaceutically acceptable vehicles or diluents. They may, for example, be administered in a form suitable for immediate release or extended release, for example, by the use of devices such as subcutaneous implants, encapsulated spheroids or osmotic pumps.

In addition to primates, such as humans, a variety of other mammals can be treated. For instance, mammals including, but not limited to, cows, sheep, goats, horses, dogs, cats, guinea pigs, rats or other bovine, ovine, equine, canine, feline, rodent or murine species can be treated.

The term "pharmaceutically acceptable" as used herein means the carrier, diluent or excipient is not deleterious to the recipient thereof.

The terms "administration of and or "administering" should be understood to mean providing to an individual in need of treatment. Administration in not included in the scope of the invention.

An individual in need of treatment may be one diagnosed with, or at risk of developing, any one of the cancers described herein.

The compositions for use of the invention, and preparations or formulations thereof may be prepared by admixing together the components of the composition, namely chymotrypsinogen and trypsinogen. The admixing may be performed sequentially or simultaneously.

The compositions for use of the invention may conveniently be presented in dosage unit form and may be prepared by any of the methods well known in the art of pharmacy. All methods include the step of bringing the active agents and protease proenzyme into association with the carrier, which constitutes one or more accessory ingredients. In general, pharmaceutical compositions are prepared by uniformly and intimately bringing the active agents and protease proenzymes into association with a liquid carrier or a finely divided solid carrier or both, and then, if necessary, shaping the product into the desired formulation. The active agents and protease proenzymes are provided in a dosage unit form in an amount sufficient to produce the desired effect upon the process or condition of diseases after single or repeated administration.

The compositions for use of the invention may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the protease proenzyme and active agent of the first and second aspects in admixture with non-toxic pharmaceutically acceptable excipients, which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated to form osmotic therapeutic tablets for control release.

Formulations for oral use may also be presented as hard gelatin capsules wherein the protease proenzyme and active agent of the first and second aspects are mixed with an inert solid diluent, for example, calcium carbonate, calcium phosphate or kaolin, or as soft gelatin capsules wherein the protease proenzyme and active agent of the first and second aspects are mixed with water or an oil medium, for example peanut oil, liquid paraffin, or olive oil.

Aqueous suspensions contain the active agent and protease proenzyme in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, n-methyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active agent and protease proenzyme in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These may be preserved by the addition of an anti-oxidant such as ascorbic acid.

Dispersible powders and granules suitable for preparation of an aqueous suspension by the addition of water provide the protease proenzyme and active agent of the first and second aspects in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present.

The compositions for use of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally- occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxy ethylene sorbitan monooleate. The emulsions may also contain sweetening and flavouring agents.

Syrups and elixirs may be formulated with sweetening agents, for example glycerol, propylene glycol, sorbitol or sucrose. They may also contain a demulcent, a preservative and flavoring and coloring agents.

The compositions for use of invention may be in the form of a sterile injectable aqueous or oleagenous suspension. This suspension may be formulated according to the known art using those suitable dispersing or wetting agents and suspending agents which have been mentioned above. The pharmaceutical compositions may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

In a particular embodiment, the compositions for use of the invention are formulated as suppositories for rectal administration of the drug. These formulations can be prepared by mixing the protease proenzyme and active agent of the first and second aspects with a suitable non-irritating excipient which is solid at ordinary temperatures but liquid at the rectal temperature and will therefore melt in the rectum to release the drug. Such materials include cocoa butter and polyethylene glycols. Rectal administration may be used to elimate entero-hepatic first pass effect in the gastrointestinal tract related to oral administration of enzymes.

The compositions for use of the invention, may also be formulated in liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolisable lipid capable of forming liposomes can be used. The liposome formulation may contain stabilisers, preservatives, excipients and the like. The preferred lipids are the phospholipids and phosphatidyl cholines, both natural and synthetic. Methods to form liposomes are known in the art.

The compositions for use of the invention may be included in a container, pack, or dispenser together with instructions for administration. The protease proenzymes and active agents, and optionally additional active agent, of the pharmaceutical composition may be provided as separated components in the container, pack, or dispenser, to be taken separately or together at the same or different time in a use or method of the invention described herein.

The following examples are provided to illustrate the invention. Any compositions that do not correspond to the definition in the present claims are examined for comparative purposes only.

## Example 1

| Material | Supplier |
|---|---|
| Dulbecco's Modified Eagle Medium (DMEM), Eagle's Minimum Essential Medium (EMEM),Minimum Essential Medium (MEM), Roswell Park Memorial Institute (RPMI) 1640 cell culture medium, Foetal Bovine Serum (FBS), GlutaMAX™, sodium bicarbonate, penicillinstreptomycin (Pen/Strep) and trypsin | Invitrogen USA (Carlsbad, CA, USA) |
| CellTiter-Blue® Cell Viability Assay | Promega (Madison, WI, USA) |
| Trypan Blue | Sigma-Aldrich (St Louis, MO, USA) |

### Cell Culture

The following table shows the growth conditions and initial cell seeding densities in cells per well used in all $IC_{50}$ determination and combination assays. The cells were cultured at 37°C in a humidified cell culture incubator supplied with 95% air/5% $CO_2$.

| Cell line | Tumor Type | Cell culture medium | Seed Density |
|---|---|---|---|
| 786-O | Kidney | RPMI+10% FBS+1% GlutaMAX™ +1% Pen/Strep | 400 |
| A27801 | Ovary | DMEM+10%FBS+1% GlutaMAX™ +1% Pen/Strep | 2500 |
| ACHN | Kidney | MEM+10%FBS+1% GlutaMAX™ +1% Pen/Strep | 4000 |

(continued)

| Cell line | Tumor Type | Cell culture medium | Seed Density |
|---|---|---|---|
| BT-474 | Breast | RPMI+10% FBS+1% GlutaMAX™ +1% Pen/Strep +1% NaHCO$_3$ | 10000 |
| C8161.9 | Melanoma | DMEM+10%FBS+1% GlutaMAX™ +1% Pen/Strep | 1500 |
| DAOY | Brain | RPMI+10% FBS+1% GlutaMAX™ +1% Pen/Strep | 1500 |
| DU 145 | Prostate | RPMI+10% FBS+1% GlutaMAX™ +1% Pen/Strep | 800 |
| G-361 | Melanoma | RPMI+10% FBS+1% GlutaMAX™ +1% Pen/Strep | 3000 |
| HCT 116 | Colorectal | RPMI+10% FBS+1% GlutaMAX™ +1% Pen/Strep | 1500 |
| HCT-15 | Colorectal | RPMI+10% FBS+1% GlutaMAX™ +1% Pen/Strep | 1500 |
| Hep3B2.1-7 | Liver | RPMI+10% FBS+1% GlutaMAX™ +1% Pen/Strep | 1500 |
| HL-60 | Leukaemia | RPMI+10% FBS+1% GlutaMAX™ +1% Pen/Strep | 50000 |
| HT-1080 | Fibrosarcoma | EMEM+10% FBS+1% GlutaMAX™ +1% Pen/Strep | 1500 |
| HT-29 | Colorectal | RPMI+10% FBS+1% GlutaMAX™ +1% Pen/Strep | 4000 |
| HuH-7 | Liver | DMEM+10% FBS+1% GlutaMAX™+1% Pen/Strep | 4000 |
| MCF-7 | Breast | RPMI+10% FBS+1% GlutaMAX™ +1% Pen/Strep | 5000 |
| MDA -MB-231 | Breast | DMEM+10% FBS+1% GlutaMAX™+1% Pen/Strep | 6000 |
| MES-SA | Uterus | RPMI+10% FBS+1% GlutaMAX™ +1% Pen/Strep | 5000 |
| NCI-H460 | Lung | RPMI+10% FBS+1% GlutaMAX™ +1% Pen/Strep | 400 |
| NCI-H82 | Lung | RPMI+10% FBS+1% GlutaMAX™ +1% Pen/Strep | 25000 |
| PC-3 | Prostate | RPMI+10% FBS+1% GlutaMAX™ +1% Pen/Strep | 2500 |
| Raji | Leukaemia | RPMI+10% FBS+1% GlutaMAX™ +1% Pen/Strep | 3000 |
| SK-OV-3 | Ovary | RPMI+10% FBS+1% GlutaMAX™ +1% Pen/Strep | 1500 |
| SNB-19 | Brain | DMEM+10% FBS+1% GlutaMAX™+1% Pen/Strep | 600 |
| U-87 MG | Brain | MEM+10% FBS+1% GlutaMAX™+1% Pen/Strep | 1500 |

All following cell lines were sourced from American Type Culture Collection (ATCC) (Rockville, MD, USA): 786-0, ACHN, BT-474, DAOY, DU 145, G-361, HCT 116, HCT-15, Hep3B2.1-7, HL-60, HT-1080, HT-29, MCF-7, MDA-MB-231, MES-SA, NCI-H460, NCI-H82, PC-3, SK-OV-3, U-87 MG.

The A2780 cell line was sourced from the National Cancer Institute (NCI) (Bethesda, MD, USA).

The C8161.9 cell line was sourced from Dr. Gavin Robertson's Laboratory (College of Medicine, Pennsylvania State University, Hershey, PA, USA)).

The HuH-7 cell line was sourced from the Japanese Collection of Research Bioresources (JCRB) Cell Bank (Osaka, Japan).

The SNB-19 and Raji cell line were sourced from the Deutsche Sammlung von Mikroorganismen und Zellkulturen (DSMZ) (German Collection of Microorganisms and Cell Cultures) (Braunschweig, Germany).

All cell lines were utilized in assays up to passage 10.

**Test Articles**

**Test Article 1**

| | |
|---|---|
| Identity: | **Trypsinogen** |
| Description: | White powder |

(continued)

| Lot Number: | 0F001644 |
| --- | --- |
| Storage Conditions: | -20°C |
| Handling Precautions: | Standard laboratory precautions |
| Manufacturer / Supplier: | Applichem (Darmstadt, Germany) / Enzyme Supplies (Oxford, UK) |

**Test Article 2**

| Identity: | **Chymotrypsinogen** |
| --- | --- |
| Description: | White powder |
| Lot Number: | 3J006510 |
| Storage Conditions: | -20°C |
| Handling Precautions: | Standard laboratory precautions |
| Manufacturer / Supplier: | Applichem (Darmstadt, Germany) / Enzyme Supplies (Oxford, UK) |

**Test Article Formulation**

Trypsinogen and Chymotrypsinogen were dissolved directly in the appropriate cell culture medium and immediately added to the cells.

**Cell Growth Assays**

The cell lines represented multiple tumour types and included: DAOY, SNB-19 and U87-MG for brain; BT-474, MDA-MB-231 and MCF-7 for breast; HCT 116, HCT-15 and HT-29 for colon; HT-1080 for fibrosarcoma; ACHN and 786-O for kidney; HL-60 and Raji for leukaemia; Hep3B2.1-7 and HuH-7 for liver; NCI-H460 and NCI-H82 for lung; C8161.9 and G-361 for melanoma; PC-3 and DU 145 for prostate; A2780 and SK-OV-3 for ovary; and MES-SA for uterus.

For combination assays, Test Articles were added to cells 24 hours post-seeding. Test Article concentrations were tested in triplicate for each cell line. Seventy-two hours post addition of Test Articles, the CellTiter-Blue® Assay was carried out on all plates.

The concentration of Trypsinogen used in the combination assays was based on the calculated $IC_{50}$ from single Test Article experiments. The concentration of Trypsinogen for each individual cell line determined the concentration of Chymotrypsinogen at the following ratios: 1:1, 1:2, 1:4, 1:6 and 1:8 (Trypsinogen:Chymotrypsinogen). Controls consisted of growth medium only and untreated cells plus growth medium (untreated control).

Assay controls used were growth medium control as a vehicle and growth-medium only (background) as opposed to phosphate-buffered saline control as vehicle and Triton-x 100 as positive control. Background subtraction was not performed for the determination of $IC_{50}$ values.

**CellTiter-Blue® Assay**

Following incubation of cells in Test Article-containing media, 10 μL of CellTiter-Blue® was added to each well, then incubated with cells for up to 6 hours. Fluorescence was measured using a Spectramax Gemini XPS Fluorometer (560 nm excitation, 590 nm emission). All data were recorded and entered into Microsoft® Excel spreadsheets for interpretation.

**Calculations**

Data collected from CellTiter-Blue® assays were plotted as dose response curves for $IC_{50}$ determination. Relative Fluorescence Units (RFU) were plotted against compound concentrations. In these plots, the X-axis (compound concentration) was represented in a logarithmic scale. $IC_{50}$ concentration was calculated as the half maximal (50%) inhibitory concentration (IC) for each compound via a variable slope curve-fitting algorithm using GraphPad Prism version 6.0e for Mac OSX (GraphPad Software, San Diego California, USA).

For combination studies, the coefficient of drug interaction (CDI) was calculated according to the following equation:

$$CDI = \frac{TC}{T \, x \, C}$$

where TC is the growth inhibition of the combination of Trypsinogen and Chymotrypsinogen, T is the growth inhibition of the single agent Trypsinogen and C the growth inhibition of the single agent Chymotrypsinogen. CDI values below 1 indicate drug synergism, whereas values above 1 indicate an antagonistic interaction of Trypsinogen and Chymotrypsinogen.

**Example 2**

**Cell Growth Inhibition by Trypsinogen and Chymotrypsinogen in Human Cancer**

Treatment with the combination of Chymotrypsinogen (C) and Trypsinogen (T) demonstrated greater growth inhibition at ratios of greater than 1:1 and less than 4:1, compared with 1:1 based on the CDI for cell lines described in Figures 1 to 18 and Tables 1 to 18. The CDI value for each combination in all cell lines tested is depicted in the Figures and Tables.

Further, the growth inhibition of liver tumour cells observed for the ratio greater than 1:1 but less than 4:1 was equal to ratios of 4:1, 6:1 and 8:1.

Further, the growth inhibition of fibrosarcoma cells was significant at all ratios tested including 2:1, 4:1, 6:1 and 8:1. The chymotrypsinogen and trypsinogen showed significant synergistic effects on growth inhibition at all ratios tested above 1:1, i.e. at 2:1,4:1,6:1 and 8:1.

Tables 1 to 18 below show the average fluorescence (relative fluorescent units - RFU) of 3 replicates, the standard error of the mean (SEM), the growth inhibition (1 = no growth inhibition, less than 1 = growth inhibition) and CDI (coefficient of drug interaction - CDI values below 1 indicate drug synergism, whereas values above 1 indicate an antagonistic interaction of Trypsinogen and Chymotrypsinogen).

**Table 1:** A2780 ovary tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 703.71 | 12.09 | - | - |
| Control (cells only) | 9659.51 | 101.65 | - | - |
| 1:1 | 3453.30 | 93.64 | 0.36 | 0.30 |
| 1:2 | 1521.3 | 36.42 | 0.16 | 0.17 |
| 1:4 | 1078.62 | 16.87 | 0.11 | 0.18 |

**Table 2:** ACHN kidney tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 711.37 | 9.82 | - | - |
| Control (cells only) | 10270.23 | 99.95 | - | - |
| 1:1 | 8766.36 | 105.03 | 0.85 | 0.93 |
| 1:2 | 7684.05 | 367.53 | 0.75 | 0.84 |
| 1:4 | 5722.51 | 116.47 | 0.56 | 0.61 |

**Table 3:** C8161.9 melanoma cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 686.77 | 7.30 | - | - |
| Control (cells only) | 7509.12 | 138.83 | - | - |
| 1:1 | 5777.01 | 128.98 | 0.77 | 0.70 |
| 1:2 | 2980.91 | 116.99 | 0.40 | 0.42 |
| 1:4 | 2088.21 | 167.84 | 0.28 | 0.31 |

**Table 4:** DAOY brain tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 664.82 | 103.85 | - | - |
| Control (cells only) | 8107.88 | 89.46 | - | - |
| 1:1 | 7637.47 | 112.26 | 0.94 | 0.84 |
| 1:2 | 3838.65 | 363.05 | 0.47 | 0.46 |
| 1:4 | 1166.91 | 18.83 | 0.14 | 0.20 |

**Table 5:** DU145 prostate tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 851.76 | 20.88 | - | - |
| Control (cells only) | 11869.06 | 447.88 | - | - |
| 1:1 | 7743.61 | 777.74 | 0.65 | 0.78 |
| 1:2 | 3562.78 | 95.25 | 0.30 | 0.53 |
| 1:4 | 2333.60 | 32.08 | 0.20 | 0.57 |

**Table 6:** G-361 melanoma cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 858.85 | 14.22 | - | - |
| Control (cells only) | 13120.01 | 103.22 | - | - |
| 1:1 | 8551.07 | 76.50 | 0.65 | 0.63 |
| 1:2 | 5499.97 | 8.27 | 0.42 | 0.49 |
| 1:4 | 2083.54 | 70.70 | 0.16 | 0.51 |

**Table 7:** HCT 116 colorectal tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 856.15 | 3.09 | - | - |

(continued)

| Treatment (ratio of T:C) | Average | SEM | Growth inhibition | CDI |
|---|---|---|---|---|
| Control (cells only) | 12280.09 | 269.70 | - | - |
| 1:1 | 12039.83 | 209.09 | 0.98 | 0.76 |
| 1:2 | 10285.43 | 225.94 | 0.84 | 0.51 |
| 1:4 | 6789.13 | 105.06 | 0.55 | 0.45 |

**Table 8:** HCT-15 colorectal tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 726.14 | 9.39 | - | - |
| Control (cells only) | 6013.01 | 321.98 | - | - |
| 1:1 | 6505.39 | 246.18 | 1.08 | 0.62 |
| 1:2 | 4612.42 | 116.92 | 0.77 | 0.48 |
| 1:4 | 3438.92 | 80.69 | 0.57 | 0.49 |

**Table 9:** Hep3B2.1-7 liver tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 696.05 | 0.75 | - | - |
| Control (cells only) | 4176.52 | 49.41 | - | - |
| 1:1 | 4005.65 | 142.07 | 0.96 | 0.78 |
| 1:2 | 3013.89 | 175.26 | 0.72 | 0.62 |
| 1:4 | 1183.40 | 14.64 | 0.28 | 0.26 |

**Table 10:** HT-1080 fibrosarcoma cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 795.80 | 18.03 | - | - |
| Control (cells only) | 10728.36 | 299.19 | - | - |
| 1:1 | 3422.98 | 75.50 | 0.32 | 0.24 |
| 1:2 | 951.95 | 17.94 | 0.09 | 0.07 |
| 1:4 | 854.90 | 1.04 | 0.08 | 0.07 |
| 1:6 | 865.40 | 9.93 | 0.08 | 0.06 |
| 1:8 | 864.92 | 4.32 | 0.08 | 0.12 |

**Table 11:** HT-29 colorectal tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 811.16 | 1.92 | - | - |
| Control (cells only) | 9701.19 | 513.03 | - | - |
| 1:1 | 9388.49 | 20.33 | 0.97 | 2.44 |
| 1:2 | 7883.19 | 127.70 | 0.81 | 0.66 |
| 1:4 | 5085.19 | 23.57 | 0.52 | 0.55 |

**Table 12:** HuH-7 liver tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 759.31 | 12.28 | - | - |
| Control (cells only) | 10328.28 | 19.12 | - | - |
| 1:1 | 3218.07 | 156.03 | 0.31 | 0.28 |
| 1:2 | 1492.25 | 15.80 | 0.14 | 0.15 |
| 1:4 | 1039.06 | 12.07 | 0.10 | 0.13 |
| 1:6 | 934.08 | 8.20 | 0.09 | 0.13 |
| 1:8 | 970.98 | 5.33 | 0.09 | 0.11 |

**Table 13:** MCF-7 breast tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 798.88 | 6.62 | - | - |
| Control (cells only) | 10769.30 | 201.72 | - | - |
| 1:1 | 11467.02 | 367.46 | 1.06 | 0.92 |
| 1:2 | 8218.43 | 182.99 | 0.76 | 0.39 |
| 1:4 | 4395.20 | 138.82 | 0.41 | 0.28 |

**Table 14:** MES-SA uterine tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 841.58 | 17.93 | - | - |
| Control (cells only) | 13440.03 | 103.85 | - | - |
| 1:1 | 12634.31 | 101.96 | 0.94 | 0.80 |
| 1:2 | 6523.11 | 254.64 | 0.49 | 0.42 |
| 1:4 | 5204.07 | 44.36 | 0.39 | 0.23 |

**Table 15:** NCI-H460 lung tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 851.99 | 6.05 | - | - |
| Control (cells only) | 12429.81 | 39.73 | - | - |
| 1:1 | 7635.43 | 375.75 | 0.61 | 0.66 |
| 1:2 | 4615.77 | 301.84 | 0.37 | 0.56 |
| 1:4 | 2633.55 | 233.71 | 0.21 | 0.38 |

**Table 16:** PC3 prostate tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 765.55 | 4.36 | - | - |
| Control (cells only) | 9759.56 | 230.34 | - | - |
| 1:1 | 6181.42 | 321.80 | 0.63 | 0.77 |
| 1:2 | 4712.59 | 45.94 | 0.48 | 0.42 |
| 1:4 | 2500.80 | 51.91 | 0.26 | 0.31 |

**Table 17:** SK-OV-3 ovary tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 686.33 | 8.43 | - | - |
| Control (cells only) | 3815.73 | 135.97 | - | - |
| 1:1 | 2357.07 | 16.78 | 0.62 | 0.64 |
| 1:2 | 1585.86 | 44.19 | 0.42 | 0.44 |
| 1:4 | 1111.47 | 53.60 | 0.29 | 0.21 |

**Table 18:** SNB-19 brainy tumour cells - Fluorescence Raw Data and Calculation of CDI and Growth Inhibition from CellTiter-Blue® Assay for Trypsinogen plus Chymotrypsinogen.

| Treatment (ratio of T:C) | Average | SEM | Growth inhibition | CDI |
|---|---|---|---|---|
| Medium only | 662.97 | 7.90 | - | - |
| Control (cells only) | 3256.44 | 45.40 | - | - |
| 1:1 | 2622.92 | 125.64 | 0.81 | 0.59 |
| 1:2 | 975.47 | 12.04 | 0.30 | 0.26 |
| 1:4 | 777.19 | 1.07 | 0.24 | 0.21 |

**Claims**

1. A composition for use in treating cancer in a subject, the composition comprising chymotrypsinogen and trypsinogen, wherein the weight ratio of chymotrypsinogen :
trypsinogen is equal to or greater than 2:1 but less than 4:1, and wherein the composition does not comprise amylase.

**2.** A composition for the use according to claim 1, wherein the weight ratio is equal to or greater than 2:1 but equal to, or less than 3:1.

**3.** A composition for the use according to claim 1 or 2, wherein the weight ratio is 2:1.

**4.** A composition for the use according to claim 1, wherein the weight ratio is 3:1.

**5.** A composition for the use according to any one of claims 1 to 4, wherein the cancer is any one of pancreatic cancer, oesophageal cancer, colon cancer, bowel cancer, prostate cancer, ovarian cancer, stomach cancer, breast cancer, liver cancer, malignant melanoma or lung cancer.

**6.** A composition for the use according to any one of claims 1 to 5, wherein the cancer is ovarian, melanoma, brain, prostate, colorectal, liver or lung.

**7.** A composition for the use according to any one of claims 1 to 6, wherein the cancer is pancreatic cancer, colon cancer or ovarian cancer, preferably wherein the cancer is pancreatic cancer.

**8.** A composition for the use of any one of claims 1 to 4, wherein the composition is for treating fibrosarcoma in a subject.

**Patentansprüche**

**1.** Zusammensetzung zur Verwendung bei der Behandlung von Krebs in einem Subjekt, wobei die Zusammensetzung Chymotrypsinogen und Trypsinogen umfasst, wobei das Gewichtsverhältnis von Chymotrypsinogen : Trypsinogen gleich oder größer als 2:1, aber kleiner als 4:1 ist, und wobei die Zusammensetzung keine Amylase umfasst.

**2.** Zusammensetzung zur Verwendung nach Anspruch 1, wobei das Gewichtsverhältnis gleich oder größer als 2:1, aber gleich oder kleiner als 3:1 ist.

**3.** Zusammensetzung zur Verwendung nach Anspruch 1 oder 2, wobei das Gewichtsverhältnis 2:1 ist.

**4.** Zusammensetzung zur Verwendung nach Anspruch 1, wobei das Gewichtsverhältnis 3:1 ist.

**5.** Zusammensetzung zur Verwendung nach einem beliebigen der Ansprüche 1 bis 4, wobei der Krebs ein beliebiger aus Bauchspeicheldrüsenkrebs, Speiseröhrenkrebs, Dickdarmkrebs, Darmkrebs, Prostatakrebs, Eierstockkrebs, Magenkrebs, Brustkrebs, Leberkrebs, malignes Melanom oder Lungenkrebs ist.

**6.** Zusammensetzung zur Verwendung nach einem beliebigen der Ansprüche 1 bis 5, wobei der Krebs Eierstock-, Melanom, Gehirn-, Prostata-, Kolorektal-, Leber- oder Lungenkrebs ist.

**7.** Zusammensetzung zur Verwendung nach einem beliebigen der Ansprüche 1 bis 6, wobei der Krebs Bauchspeicheldrüsenkrebs, Dickdarmkrebs oder Eierstockkrebs ist, vorzugsweise wobei der Krebs Bauchspeicheldrüsenkrebs ist.

**8.** Zusammensetzung zur Verwendung nach einem beliebigen der Ansprüche 1 bis 4, wobei die Zusammensetzung zur Behandlung eines Fibrosarkoms in einem Subjekt ist.

**Revendications**

**1.** Composition destinée à être utilisée dans le traitement du cancer chez un sujet, la composition comprenant du chymotrypsinogène et du trypsinogène, où le rapport pondéral chymotrypsinogène:trypsinogène est égal ou supérieur à 2:1 mais inférieur à 4:1, et où la composition ne comprend pas d'amylase.

**2.** Composition destinée à être utilisée selon la revendication 1, où le rapport pondéral est égal ou supérieur à 2:1 mais égal ou inférieur à 3:1.

**3.** Composition destinée à être utilisée selon la revendication 1 ou 2, où le rapport pondéral est 2:1.

**4.** Composition destinée à être utilisée selon la revendication 1, où le rapport pondéral est 3:1.

**5.** Composition destinée à être utilisée selon l'une quelconque des revendications 1 à 4, où le cancer est l'un quelconque parmi le cancer du pancréas, le cancer de l'oesophage, le cancer du côlon, le cancer de l'intestin, le cancer de la prostate, le cancer des ovaires, le cancer de l'estomac, le cancer du sein, le cancer du foie, le mélanome malin ou le cancer du poumon.

**6.** Composition destinée à être utilisée selon l'une quelconque des revendications 1 à 5, où le cancer est de l'ovaire, un mélanome, du cerveau, de la prostate, colorectal, du foie ou du poumon.

**7.** Composition destinée à être utilisée selon l'une quelconque des revendications 1 à 6, où le cancer est le cancer du pancréas, le cancer du côlon ou le cancer de l'ovaire, de préférence où le cancer est le cancer du pancréas.

**8.** Composition destinée à être utilisée selon l'une quelconque des revendications 1 à 4, où la composition est pour traiter le fibrosarcome chez un sujet.

**Figure 1**

| CDI | 0.30 | 0.17 | 0.18 |

**Figure 2**

**Figure 3**

| CDI | 0.70 | 0.42 | 0.31 |

**Figure 4**

**Figure 5**

| CDI | 0.78 | 0.53 | 0.57 |

**Figure 6**

**Figure 7**

| | | | |
|---|---|---|---|
| CDI | 0.76 | 0.51 | 0.45 |

**Figure 8**

| | CDI | 0.62 | 0.48 | 0.49 |
|---|---|---|---|---|

**Figure 9**

| | Control | 1:1 | 1:2 | 1:4 |
|---|---|---|---|---|
| CDI | | 0.78 | 0.62 | 0.25 |

**Figure 10**

| CDI | 0.24 | 0.07 | 0.07 | 0.06 | 0.12 |

## Figure 11

| CON | 2.44 | 0.66 | 0.55 |

**Figure 12**

| CDI | 0.28 | 0.15 | 0.13 | 0.13 | 0.11 |

Figure 13

| CDI | 0.92 | 0.39 | 0.28 |

**Figure 14**

| CDi | 0.80 | 0.42 | 0.23 |

**Figure 15**

| CDI | 0.66 | 0.56 | 0.38 |

**Figure 16**

**Figure 17**

| CDI | 0.64 | 0.44 | 0.21 |

**Figure 18**

| CDI | 0.59 | 0.26 | 0.21 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011047434 A
- WO 2017127892 A